# EUROPEAN PATENT SPECIFICATION

(11) **EP 2 488 216 B1**
(45) Date of publication and mention of the grant of the patent: **21.12.2016**
(21) Application number: 10763732.4
(22) Date of filing: 15.10.2010
(51) Int. Cl.: A61K 35/62, A61L 2/18, A61P 31/00

(54) **EXTRACT OF LEECHES AS ANTI-BACTERIAL AGENT**
BLUTEGELEXTRAKTE ALS ANTIBAKTERIELLES MITTEL
EXTRAIT DE SANGSUES COMME AGENT ANTIBACTÉRIEN

(30) Priority: 15.10.2009 EP 09290786
(43) Date of publication of application: 22.08.2012
(73) Proprietor: Centre National de la Recherche Scientifique (C.N.R.S.), 75794 Paris Cedex 16 (FR); Université de Lille 1 Sciences et Technologies, 59655 Villeneuve d'Ascq Cedex (FR)
(72) Inventor: TASIEMSKI, Aurélie, F-59110 La Madeleine (FR); SALZET, Michel, F-59830 Bourghelles (FR)
(74) Representative: Grosset-Fournier, Chantal Catherine
(86) International application number: PCT/EP2010/065563
(87) International publication number: WO 2011/045427

(56) References cited:
- WO-A1-90/12808
- WO-A1-92/07005
- FR-A1- 2 843 883
- GB-A- 2 300 190
- US-A- 5 710 131
- MICHALSEN A ET AL: "Effectiveness of leech therapy in women with symptomatic arthrosis of the first carpometacarpal joint: A randomized controlled trial", PAIN, ELSEVIER SCIENCE PUBLISHERS, AMSTERDAM, NL, vol. 137, no. 2, 15 July 2008 (2008-07-15) , pages 452-459, XP022765585, ISSN: 0304-3959, DOI: DOI:10.1016/J.PAIN.2008.03.012 [retrieved on 2008-04-14]
- DATABASE WPI Week 200479 Thomson Scientific, London, GB; AN 2004-802994 XP002614249, & RU 2 238 713 C2 (NIKONOV G I) 27 October 2004 (2004-10-27)

## Description

The present invention relates to use of extract of leeches.

Today, the infection by resistant bacteria is a more and more serious public sanitary problem. Many bacterial lines became resistant to most kinds of currently disposable antibiotic treatments. It becomes a real challenge to find new antibacterial agents to fight against resistant bacteria, particularly in the frame of fighting against nosocomial infections. The market for new antibacterial agents is estimated to be 5 billion dollars in US.

In industrial domain, elimination of bacteria is effected, up to now, by long, heavy and expensive processes, such as cleaning operation and large scale disinfection. However, said processes are sometimes not efficient. Furthermore, since more and more drastic norms, such as norms "REACH" or "BIOCIDES", enter in force, the number of usable products considerably decrease. For example, FDA has recently limited the use of certain components, such as Triclosan, that generates important bacterial resistance. Furthermore, most of antibacterial agents used up to now are not natural and able to provoke environmental problems, linked to the difficulty of elimination of these agents.

Food infection is another public healthy problem linked to the contamination of the food by pathogenic bacteria such as *Salmonellae, Listeria monocytogenes, Staphylococcus aureus, Bacillus cereus, Enterococcus fecalis* or *Campylobacter jejuni.* The incidence of food infection, that is frequent in the developing countries, is also increasing in the developed countries.

Recently, researchers have turned to new biological source based on new mechanisms, and subsequently several new antibacterial agents that would not provoke bacterial resistance have been found.

The antimicrobial peptides (AMP) is a kind of natural antibiotic, not immunogenic and produced by plants or animals having a special mode of action and activity. They generally react at level of bacterial membranes and do not favor the apparition of resistant bacterial lines. Among the AMP present in the market, *recombinant plectasin,* for example, is used for fighting against the Gram+ bacteria, particularly against *Streptococcus pneumonia,* a kind of bacteria known for its resistance to classic antibiotherapy. Other AMPs, such as the *Pexiganan,* are today in clinical phase 3. Said compound, derived from magainin of amphibians, targets the treatment of impetigo and stomach ulcer. Other compoounds may be cited like *isaganan,* derived from porcine protegrin for the treatment of oesophagitis or stomach ulcer, or *Neuprex* issued from human protein rBP123, or still *Ominagan,* issued from indolicin, for the treatment of infection linked to the use of catheters.

The history of use of leeches of species *Hirudo medicinalis* in medicine goes back to the 18^{th} Egyptian dynasty. In the 19^{th} century in France, leeches have been used in the treatment of pharyngitis, ophthalmic problems, obesity, and mental disorders. During the treatment, leeches were directly placed on the abdomen of patient. Later, the treatment by leeches was failing into disuse until the fifties of the last century. For example, in 1948, leeches were used by P.Durand, P.Viard and R. Vendel in the treatment of asthma.

Today, with the development of microsurgery, leeches are generally used in hospitals during a plastic or traumatological surgery to resolve many problems due to the insufficiency of venous drainage. The survival of a reimplanted tissue depends on the efficiency of venous return. However, it is very difficult to establish a venous return since it is more difficult to stitch a vein than an artery. Certain surgeons then consider local application of leeches to resolve this problem. By their suction, leeches stimulate drainage of tissue in danger of necrosis. They favor also the restoration of capillaries match between the faces of a wound where a surgical surture is technically impossible.

In 2003, German researchers found that leech therapy can help relieve symptoms in patients with osteoarthritis of the knee by directly placing leeches on the knees of patients (Michalsen et al., Ann intern Med. 2003; 139: 724-730).

However, leech therapy used up to now, using living organisms, presents a potential infection risk, especially when there is an enter gate, such as a leech bite. In 1983, Whitlock et al. (Br J Plast Surg 1983; 36: 240-3) suggested for the first time the possibility of subcutanous infection caused by *Aeromonas hydrophila,* a kind of Gram negative aerobic bacteria, by the intermediate of leech therapy. This kind of bacteria exists in intestinal flora of leeches and digests the red blood cells taken by leeches. This bacterium is also linked to two types of gastroenteritis. The first type is a disease similar to cholera, which causes rice-water diarrhea. The other type of disease is dysenteric gastroenteritis, which causes loose stools filled with blood and mucus.

*Aeromonas hydrophila* is not the only pathogen presents in leeches that can provoke an infection. A small quantity of Gram negative bacillus including *Pseudomonas* and a tiny proportion of anaerobic germ can also be found on the surface of leeches.

Disinfection of leeches before leech therapy is generally effected by immersion of leeches in antibiotic solution and then rinsing by sterile water. However, this method can not always efficiently disinfect leeches. Sometimes, it causes also allergy in patient and affects the suction capacity of leeches.

The patent application FR2834292 reports the use of leeches extract as anticoagulant, antiinflammatory or thrombolytic agents.

The patent application FR2834293 presents the use of extract prepared from starved leeches as antinarcotic, antistress or antiinflammatory agent.

The patent application FR2843883 concerns non-irritating leech extract, having antiinflammatory and/or antipsoriatic activity, obtained by suspending homogenized crude extract in saline, incubating and recovering supernatant.

In summary, these prior patent applications provide only information about antiinflammatory and anticoagulant activity of leeches extract, in particular saliva extract.

However, there is still an important need to find a new, safe, natural antibacterial agent that on the one hand does not provoke bacterial resistance and on the other hand does not have the drawback relating to the operation of living organisms.

Recently, in the frame of studies relating to the development of diagnostic tools for osteoarthritis, the Inventors of the present invention have surprisingly found that osteoarthritis has a bacterial origin, and discovered that leeches extract exposes high anti-bacterial property. In the previous works, the Inventors have found 4 new antimicrobial peptides in central nervous system of leeches. Now, the Inventors find that antibacterial agents are also present in leeches' saliva.

The first aspect of the present invention relates to leeches extract for its use in the treatment of bacterial diseases.

According to the present invention, leeches extract can be obtained from homogenised tissues from the whole leech as well as any part of the leech, in particular from salivary glands, said homogenised tissues showing antibacterial activity.

Leeches used in the present invention can be any hematophagous leeches, in particular the Hirudinidea *e.g. Hirudo medicinalis*, *Hirudo verbena, Hirudinaria mallinensis*, *Haemopis marmorata, Macrobdella decora.*

In an advantageous embodiment, the leeches extract is for use in the treatment of diseases of the group comprising bacterial origin arthritis, foodborne illnesses caused by a pathogenic bacteria or nosocomial infections.

According to the invention, bacterial origin arthritis comprises septic arthritis.

According to the present invention, foodborne illnesses mean food infection caused by the presence of bacteria, for example, *Campylobacter jejuni, Salmonellas, Listeria mohocytogenes, Staphylococcus aureus, Bacillus cereus, Enterococcus fecalis,* or other microbes which infect the body after consumption of food.

According to the present invention, nosocomial infections mean infections which are the result of treatment in a hospital or a healthcare service unit, but secondary to the patient's original condition. Infections are considered nosocomial if they first appear 48 hours or more after hospital admission or within 30 days after discharge. This type of infection is also known as a hospital-acquired infection (or more generically healthcare-associated infection).

Nosocomial infections are often caused by Gram-positive bacteria, such as *Staphylococcus aureus,* Methicillin Resistant *Staphylococcus aureus, Clostridium difficile,* Vancomycin-resistant *Enterococcus,* or Gram-negative bacteria, such as *Pseudomonas aeruginosa, Acinetobacter baumannii, Legionella,* or mycobacteria. As nosocomial infections, we can recite as example without limitation, ventilator associated pneumonia, hospital-acquired tuberculosis, hospital-acquired urinary tract infection, hospital-acquired pneumonia or hospital-acquired gastroenteritis.

In another advantageous embodiment, the leeches extract of the present invention is for use in the treatment of diseases caused by bacteria belonging to biological agents of class II or class III.

The biological agents are classified in 4 classes according to the importance of risk of propagation that they represent.
Class I is the biological agent which is not susceptible to provoke a disease in human.
Class II is the biological agent which can provoke a disease in human and constitute a danger for the public, but which propagation in the community is improbable. There is generally a prophylaxis or an efficient treatment for this disease.
Class III is the biological agent which can provoke a disease in human and constitute a serious danger for the public, and which has a risk of propagation in community. However there is generally a prophylaxis or an efficient treatment for this disease.
Class IV is the biological agent which provokes diseases in human and constitutes a serious danger for the pubic, and which has a high risk of propagation in community. There is not generally any prophylaxis or efficient treatment for these diseases.

In a more advantageous embodiment, bacteria of class II or class III are selected from the group consisting of *Acinetobacter thyphimirium, Salmonella thyphimirium, Alcaligenes faealis, Haemophillus influenzae, Shewanella, Escherichia coli, Aerococcus viridans,*

*Microcuccus luteus, Staphylocuccus*, *saprophyticus.*

Another aspect of the invention is the use of leeches extract as a cleaning product.

In an advantageous embodiment, said cleaning product is selected from the group consisting of a hospital disinfectant product or a general household disinfectant such as a window cleaner, a bathroom cleaner, a kitchen cleaner, a floor cleaner, a laundry detergent, a cleaning supply, a fruit and a vegetable wash, and a fabric softener.

Another aspect of the present invention is the use of leeches extract as anti-bacterial agent in a cosmetic composition for use in the treatment of bacterial diseases.

In an advantageous embodiment, the leeches extract used in the present invention is a leeches saliva extract. According to the invention an extract of saliva means crude saliva or purified saliva.

Saliva of leeches may be extracted according to any conventional method, for example, the method of Bligh. E.J. and Dyer. H.J. (Biochem, physiol. 37, 911-917 (1959)).

In a more advantageous embodiment, the leeches extract used in the present invention is a purified leeches extract.

Purified leeches saliva extract may be obtained according to any conventional process, for example by the following method:
- extract leeches saliva in large quantity according to a conventional method,
- sterilize leeches saliva by any conventional method, such as by UV, and optionally
- purify leeches saliva by any conventional method, such as by chromatography.

Another aspect of the invention concerns a pharmaceutical composition comprising leeches saliva extract for its use in the treatment of bacterial diseases.

In an advantageous embodiment, the pharmaceutical composition is used for the treatment of diseases selected from the group comprising septic arthritis, or foodborne illnesses caused by a pathogenic bacteria or nosocomial infections.

In a more advantageous embodiment, the nosocomial infections are caused by a kind of bacteria selected from the group consisting of bacteria (gram +, gram -) or mycobacteria.

In another more advantageous embodiment, the pharmaceutical composition acts against the bacteria of Class II and/or of Class III.

In a particularly more advantageous embodiment, the bacteria is selected from the group consisting of *Acinetobacter thyphimirium, Salmonella thyphimirium, Alcaligenes faealis, Haemophillus influenzae, Shewanella, Escherichia coli, Aerococcus viridans, Microcuccus luteus, Staphylococcus, saprophyticus, Helicobacter pyroli*.

According to the invention, the pharmaceutical composition can comprise, besides of leeches extract, any other active substances, for example an anti-inflammatory agent, or other antibacterial agents.

The anti-inflammatory agent comprised in said pharmaceutical composition can be an anti-inflammatory agent known to one skilled in the art, such as steroids of synthetic or natural origin, non steroidal anti-inflammatory agent, and an anti-inflammatory peptides from vegetable or animal origin, such as from leeches.

The antibacterial agents can also be any agent known from the art.

According to the present invention, the pharmaceutical composition can also contain a pharmaceutical acceptable carrier, or diluents, emulsifiers, adjuvants and vehicles as desired.

According to the present invention, the pharmaceutical composition may be administrated by topical route, enteral route or parenteral route by injection or infusion.

According to the present invention, the pharmaceutical composition can be manufactured in a liquid form, such as an injection, a spray, or in a solid form, such as a cream, patches, tablets, capsules.

Another object of the disclosure is a method for treating or preventing diseases selected from foodborne illnesses caused by a pathogenic bacteria or nosocomial infections, said method comprising administration of an extract of leeches to a patient in need thereof.

The invention is illustrated by the following example.

### Example

### Test of antimicrobial activity of crude extract of leeches' saliva

50µl of crude extract of leeches' saliva are added to 500µl of culture of different bacteria in exponential phase. The tested bacteria are listed in the following table. The bactericidal activity of crude extract of leeches' saliva in different bacterial solution is tested during 24 hours. When activity was detected, all the bacteria are killed in less than 24 hours.

| **Gram-negative bacteria** | |
|---|---|
| *Acinetobacter baumannii* | ++ |
| *Salmonella thyphimirium* | + |
| *Alcaligenes faecalis* | + |
| *Haemophillus influenzae* | + |
| *Shewanella* | ++ |
| *Echerichia coli* | + |
| | |

| **Gram-positive bacteria** | |
|---|---|
| *Aerococcus viridans* | ++ |
| *Micrococcus luteus* | ++ |
| *Staphylococcus saprophyticus* | + |
| *Staphylococcus aureus* | + |

| | |
|---|---|
| *(*++*) high activity detected;(*+*) low activity detected* | |

## Claims

1. Leeches extract for use in the treatment of bacterial diseases.

2. Leeches extract for use according to claim 1, wherein the bacterial disease is selected from the group comprising septic arthritis, foodborne illnesses caused by a pathogenic bacteria and nosocomial infections.

3. Leeches extract for use according to claim 2, wherein the nosocomial infection is caused by bacteria selected from the group consisting of bacteria (gram +, gram -) or mycobacteria.

4. Leeches extract for use according to any one of claims 1 to 3, wherein the bacteria are the bacteria of Class II and/or of Class III.

5. Leeches extract for use according to claim 4, wherein said bacterium is selected from the group consisting of *Acinetobacter thyphimirium, Salmonella thyphimirium, Alcaligenes faealis, Haemophillus influenzae, Shewanella, Escherichia coli, Aerococcus viridans, Micrococcus luteus*, *Staphylococcus, saprophyticus, Helicobacter pylori.*

6. Leeches extract for use according to any one of claims 1 to 5, wherein said leeches extract is a saliva leeches extract.

7. Leeches extract for use according to claim 6, wherein said leeches extract is a purified leeches extract.

8. Leeches extract for use according to any one of claims 1 to 7, wherein said leeches extract is extracted from the leech species chosen from the Hirudinidea, particularly form *Hirudo medicinalis*, *Hirudo verbena, Hirudinaria mallinensis, Haemopis marmorata*, *Macrobdella decora.*

9. Use of extract of leeches as a cleaning product, selected from the group comprising a hospital disinfectant, a general household disinfectant, a window cleaner, a bathroom cleaner, a kitchen cleaner, a floor cleaner, a laundry detergent, a cleaning supply, a fruit and a vegetable wash.

10. Extract of leeches for its use as anti-bacterial agent in a cosmetic composition for use in the treatment of bacterial diseases.

11. Use or extract for use according to claims 9 to 10, wherein said extract of leeches is an extract of saliva of leeches.

12. Pharmaceutical composition comprising leeches saliva extract, for use in the treatment of bacterial diseases.

13. Pharmaceutical composition for use according to claim 12, wherein the bacterial disease is selected from the group comprising septic arthritis, foodborne illnesses caused by a pathogenic bacteria or nosocomial infections.

14. Pharmaceutical composition for use according to claim 13, wherein said pharmaceutical composition is administrated by topical route, enteral route or parenteral route by injection or infusion.

## Patentansprüche

1. Blutegelextrakt zur Verwendung bei der Behandlung von bakteriellen Krankheiten.

2. Blutegelextrakt zur Verwendung nach Anspruch 1, wobei die bakterielle Krankheit ausgewählt ist aus der Gruppe enthaltend septische Arthritis, durch pathogene Bakterien verursachte Lebensmittelvergiftungen und nosokomiale Infektionen.

3. Blutegelextrakt zur Verwendung nach Anspruch 2, wobei die nosokomiale Infektion durch Bakterien ausgewählt aus der Gruppe bestehend aus Bakterien (gram-positiv, gram-negativ) oder Mykobakterien verursacht wird.

4. Blutegelextrakt zur Verwendung nach einem der Ansprüche 1 bis 3, wobei die Bakterien die Bakterien von Klasse II und/oder von Klasse III sind.

5. Blutegelextrakt zur Verwendung nach Anspruch 4, wobei das Bakterium ausgewählt ist aus der Gruppe bestehend aus *Acinetobacter thyphimirium, Salmonella thyphimirium, Alcaligenes faealis, Haemophillus influenzae, Shewanella, Escherichia coli, Aerococcus viridans*, *Microcuccus luteus, Staphylocuccus saprophyticus, Helicobacter pylori.*

6. Blutegelextrakt zur Verwendung nach einem der Ansprüche 1 bis 5, wobei der Blutegelextrakt ein Extrakt aus Blutegelspeichel ist.

7. Blutegelextrakt zur Verwendung nach Anspruch 6, wobei der Blutegelextrakt ein aufgereinigter Blutegelextrakt ist.

8. Blutegelextrakt zur Verwendung nach einem der Ansprüche 1 bis 7, wobei der Blutegelextrakt aus den Blutegelspezies ausgewählt aus den Hirudinidae extrahiert ist, insbesondere aus *Hirudo medicinalis, Hirudo verbena, Hirudinaria mallinensis, Haemopis marmorata, Macrobdella decora.*

9. Verwendung von Blutegelextrakt als Reinigungsprodukt, ausgewählt aus der Gruppe enthaltend ein Krankenhausdesinfektionsmittel, ein allgemeines Haushaltsinfektionsmittel, ein Fensterputzmittel, einen Badreiniger, einen Küchenreiniger, einen Fußbodenreiniger, ein Waschmittel, ein Putzmittel, ein Obst- und Gemüsewaschmittel.

10. Verwendung von Blutegelextrakt als antibakterielles Mittel in einer kosmetischen Zusammensetzung zur Verwendung bei der Behandlung von bakteriellen Krankheiten.

11. Verwendung oder Extrakt zur Verwendung nach den Ansprüchen 9 bis 10, wobei der Blutegelextrakt ein Extrakt aus Blutegelspeichel ist.

12. Pharmazeutische Zusammensetzung, welche einen Extrakt aus Blutegelspeichel zur Verwendung bei der Behandlung von bakteriellen Krankheiten enthält.

13. Pharmazeutische Zusammensetzung zur Verwendung nach Anspruch 12, wobei die bakterielle Krankheit ausgewählt ist aus der Gruppe enthaltend septische Arthritis, durch pathogene Bakterien verursachte Lebensmittelvergiftungen oder nosokomiale Infektionen.

14. Pharmazeutische Zusammensetzung zur Verwendung nach Anspruch 13, wobei die pharmazeutische Zusammensetzung über einen topischen Weg, einen enteralen Weg oder einen parenteralen Weg durch Injektion oder Infusion verabreicht wird.

## Revendications

1. Extrait de sangsue pour son utilisation dans le traitement de maladies bactériennes.

2. Extrait de sangsue pour son utilisation selon la revendication 1, dans laquelle la maladie bactérienne est sélectionnée dans le groupe comprenant l'arthrite septique, les maladies d'origine alimentaires causées par une bactérie pathogène et les infections nosocomiales.

3. Extrait de sangsue pour son utilisation selon la revendication 2, dans laquelle l'infection nosocomiale est causée par des bactéries sélectionnées dans le groupe consistant en des bactéries (gram +, gram -) ou des mycobactéries.

4. Extrait de sangsue pour son utilisation selon l'une quelconque des revendications 1 à 3, dans laquelle les bactéries sont les bactéries de classe II et/ou de classe III..

5. Extrait de sangsue pour son utilisation selon la revendication 4, dans laquelle ladite bactérie est sélectionnée dans le groupe consistant en *Acinetobacter thyphimirium, Salmonella thyphimirium, Alcaligenes faealis, Haemophillus influenzae, Shewanella, Escherichia coli, Aerococcus viridans, Microcuccus luteus, Staphylocuccus, saprophyticus, Helicobacter pylori.*

6. Extrait de sangsue pour son utilisation selon l'une quelconque des revendications 1 à 5, dans laquelle l'extrait de sangsue est un extrait de salive de sangsue.

7. Extrait de sangsue pour son utilisation selon la revendication 6, dans laquelle l'extrait de sangsue est un extrait de sangsue purifié.

8. Extrait de sangsue pour son utilisation selon l'une des revendications 1 à 7, dans laquelle ledit extrait de sangsue est extrait d'une espèce de sangsue choisie parmi les Hirudinidea, en particulier parmi *Hirudo medicinalis, Hirudo verbena, Hirudinaria mallinensis, Haemopis marmorata, Macrobdella decora.*

9. Utilisation d'extrait de sangsue comme produit de nettoyage, sélectionné dans le groupe comprenant un désinfectant hospitalier, un désinfectant domestique général, un produit nettoyant pour vitres, un produit nettoyant pour salles de bain, un produit nettoyant pour cuisines, un produit nettoyant pour sols, un détergent à lessive, un produit de nettoyage, un produit nettoyant pour fruits et légumes.

10. Extrait de sangsue pour son utilisation comme agent antibactérien dans une composition cosmétique pour son utilisation dans le traitement de maladies bactériennes.

11. Utilisation ou extrait pour son utilisation selon les revendications 9 à 10, dans laquelle ledit extrait de sangsue est un extrait de salive de sangsue.

12. Composition pharmaceutique comprenant un extrait de salive de sangsue, pour son utilisation dans le traitement de maladies bactériennes.

13. Composition pharmaceutique pour son utilisation selon la revendication 12, dans laquelle la maladie bactérienne est sélectionnée dans le groupe comprenant l'arthrite septique, les maladies d'origine alimentaires causées par une bactérie pathogène ou les infections nosocomiales.

14. Composition pharmaceutique pour son utilisation selon la revendication 13, dans laquelle la composition pharmaceutique est administrée par voie topique, par voie entérale ou par voie parentérale par injection ou perfusion.
